# EUROPEAN PATENT SPECIFICATION

(11) **EP 3 647 403 B1**
(45) Date of publication and mention of the grant of the patent: **07.04.2021**
(21) Application number: 18204112.9
(22) Date of filing: 02.11.2018
(51) Int. Cl.: C12M 1/00, B01J 19/12, F21S 4/28

(54) **LED ARRANGEMENT FOR USE IN A BIOREACTOR**
LED-ANORDNUNG ZUR VERWENDUNG IN EINEM BIOREAKTOR
AGENCEMENT DE DEL DESTINÉ À ÊTRE UTILISÉ DANS UN BIORÉACTEUR

(43) Date of publication of application: 06.05.2020
(73) Proprietor: O&N B.V., 8701 DV Bolsward (NL)
(72) Inventor: VAN VELZEN, Dick, 8711 JD WORKUM (NL); HULSHOFF, Hendrik Jan, 8701 DV BOLSWARD (NL); MULDER, Gerardus Rudi Maria, 8701 BL BOLSWARD (NL); OOSTERBAAN, Wiebe IJsbrand Leo, 8702 CA BOLSWARD (NL); STEL, Jarno, 9697 ME BLIJHAM (NL); OELEN, Jan Johannes, 7963 RP RUINEN (NL); KOEK, Charles Adrianus Gerardus, 8651 EB IJLST (NL); MAESSEN, Theodorus Antonius, 6096 CH GRATHEM (NL); VAN DRUNEN, Rudi, 2341 PH OEGSTGEEST (NL)
(74) Representative: van Dam, Vincent

(56) References cited:
- WO-A1-2013/138690
- CN-A- 101 629 142
- DE-A1-102014 012 217

## Description

This invention relates to a carrier device for LED arrays, a LED lamp comprising this device and a bioreactor comprising this lamp. The invention also relates to a method for growing organisms capable of photosynthesis in an aqueous liquid wherein the LED lamp is used.

Organisms capable of photosynthesis convert light energy into chemical energy by converting carbon dioxide and water into the end-products oxygen and carbohydrates. This process is energized by light.

Algae form an example of such organisms, and are well-known as efficient producers of biomass. During photosynthesis, algae utilize carbon dioxide and light in the presence of water to produce oxygen and biomass. The algae produce lipids, vegetable oils and proteins which can be used for various purposes, for example as a source for human or animal nutrition or biofuel.

Algae grow best under controlled conditions. For instance, algae are sensitive to temperature and light conditions. Algal yield may be improved by controlling the growth parameters, such as temperature, CO₂ levels, light and nutrients.

Various methods have been used to grow algae but it appears difficult to grow algae efficiently on a commercial scale.

It has been described to grow algae in bioreactors using LED lamps as the light source. For purposes of algal growth the LED lamps can be submerged in the algal growth medium. LED lamps have the advantage that they offer the versatility to accommodate light requirement variations amongst various algal species. LEDs are also safer and more efficient than traditional lighting sources.

The use of LED light sources for algal growth also has some disadvantages which are associated to the fact that, apart from light, LEDs produce also heat. In fact, most of the electricity in a LED is converted to heat rather than light (∼ 50-60% heat: 40-50% light). This heat production has detrimental effects on the efficiency of the bioreactor used for growth of the algae.

DE 10 2014 012217 A1 discloses a lamp module comprising light-emitting diodes and a photoreactor.

When LEDs are used in a bioreactor for algae, it is in general preferred that LEDs are encapsulated by a housing of light transmitting material, which housing is submerged in the algal growth medium. Because of the heat produced by the LEDs the light transmitting material of the housing is also heated. This causes caking of algae onto the surface of the housing, obscuring the LEDs from the growing algae and thus lowering the efficiency of the reactor.

Furthermore, local heat differences in the growth medium caused by heat production of the LEDs may cause stress to the growing algae, leading to inefficient growth.

The present invention aims to overcome these disadvantages by the provision of a device for holding LED arrays which allows the cooling of the LEDs in an efficient manner, while it is at the same time suitable for use in a bioreactor for growing organisms capable of photosynthesis, such as algae.

### Summary of the invention

In a first aspect the invention relates to a carrier device for LED arrays according to claim 1.

In a second aspect the invention relates to a LED lamp comprising the device of the first aspect of the invention, and at least one LED array arranged at the outer surface of each outer wall, wherein most of the length of the outer walls is covered by said LED arrays.

In a third aspect the invention relates to a bioreactor for growing organisms capable of photosynthesis in an aqueous liquid, comprising a tank for accommodating the aqueous liquid with said organisms in it; and a plurality of LED lamps of the second aspect of the invention, wherein the LED lamps are at least partially submerged in said aqueous liquid.

In a fourth aspect the invention relates to a method for growing organisms capable of photosynthesis in an aqueous liquid, comprising providing a flow of said aqueous liquid, and exposing the organisms in the flowing aqueous liquid to a plurality of LED lamps of the second aspect of the invention, wherein the LED lamps are at least partially submerged in said aqueous liquid.

The inventors have discovered that the triangular arrangement of the carrier device which enables that in a LED lamp according to the invention the longitudinal axes of the LED arrays or rows of LEDs of different outer walls are spaced apart 120°, enables optimal cooling of the LED lamps mounted on the carrier device of the invention. When LED arrays mounted to the carrier device of the invention - i.e. LED lamps according to the invention - are used in a bioreactor for growing organisms capable of photosynthesis, such as algae, this way the problem of caking of these organisms onto the surface of the housing of said LEDs is reduced to a minimum, thus improving the efficiency of the reactor. Furthermore, because of the effective cooling as a result of the equilateral triangular arrangement of the carrier device of the invention, local heat differences in the growth medium caused by heat production of the LEDs are also reduced to a minimum, which also contributes to growth efficiency of said organisms. As such the bioreactor according to the invention and the method according to the invention, which apply the carrier device and LED lamps of the invention are more effective than prior art methods and bioreactors for growing organisms capable of photosynthesis.

### Short description of the figures

Fig. 1 shows a schematic representation of an embodiment of the carrier device of the invention.
Figs. 2-4 shows a schematic representation of an embodiment of the LED lamp of the invention in different stages of its mounting.
Fig. 5 shows a schematic representation of a cross section in the longitudinal direction at the position of line v of an embodiment of the LED lamp of Fig. 4.
Figs. 6 and 7 show a schematic representation of an embodiment of the LED lamp of the invention in a tubular housing.
Fig. 8 shows a schematic representation of an embodiment of the bioreactor of the invention.
Fig. 9 shows a cross section along line B of the bioreactor of Fig. 8.
Fig. 10 shows a cross section along line A of the bioreactor of Fig. 8 in case the tank of the bioreactor is filled with liquid growth medium.

### Detailed description of the invention

The present invention provides a carrier device for holding LED arrays. It is particularly suitable for use in an aqueous medium containing growing organisms capable of photosynthesis. When used as part of a LED lamp this carrier contributes to optimal temperature control of the growth medium and prevention of caking of dead algae onto the light source.

The key compartment of the carrier device according to the invention is a holder with a first end and a second end and three elongated outer walls extending between the first and second end, wherein each wall is laterally connected to the other two walls by a water impermeable connection, and wherein the walls are oriented with respect to each other to define a space of substantially equilateral triangular cross section. By using the term "substantially" it is meant that small deviations are possible, for instance the contact angles between walls may not be perfectly sharp, for instance because of welding or glueing. What is important is that a perpendicular line with respect to the wall surface of an outer wall is in a 120° configuration with respect to a perpendicular line with respect to the wall surface of the neighbouring wall. Within said space of substantially equilateral triangular cross section first and second hollow compartments are provided, which serve, when in operation, as a passage for a cooling liquid to effect cooling of LEDs mounted onto the holder. It is crucial that each wall is laterally connected to the other two walls by a water impermeable connection, because otherwise, when the carrier device is used in a LED lamp arrangement in a bioreactor, cooling liquid would be able to leak out of the first and second hollow compartments. The LEDs thus are not in direct contact with the cooling liquid.

It is preferred that the first and second hollow compartment are defined by an inner wall extending between said first and said second end and extending perpendicular from the inner surface of one of the outer walls to the opposite inner angle position between the other outer walls, wherein the connection between said inner wall and said inner surface and inner angle position is water impermeable. In other words, said inner wall separates the first and second compartment. This way two compartments of equal volume are provided.

At said first end a first closing means is provided and at said second end a second closing means is provided, wherein said first closing means comprises an inlet for introducing a cooling liquid into said first hollow compartment, and an outlet for passing said cooling liquid out of said second hollow compartment; and wherein at the second end of said holder one or more channels are provided which allow flow of said cooling liquid between the first hollow compartment and the second hollow compartment, i.e. from the first hollow compartment to the second hollow compartment.

It is preferred that screw threads are provided in the inner wall of the holder at the first and second end of the holder. This makes it possible to attach and fasten closing means, such as end caps onto the first and second end in a convenient way. Moreover, this way the closing means can be removably attached to the holder which is advantageous for maintenance purposes or for replacement of LED arrays.

As mentioned above, the first closing means comprises an inlet for introducing a cooling liquid into said first hollow compartment, and an outlet for passing said cooling liquid out of said second hollow compartment. This allows to connect an inlet tube to the first opening for introducing cooling liquid from a cooling liquid source and an outlet tube to the second opening for passing said cooling liquid away from the device of the invention.

At the second end of the holder one or more channels are provided which allow flow of a cooling liquid from the first hollow compartment to the second hollow compartment.

In one embodiment this/these channel(s) at the second end of said holder is/are an opening or openings in said inner wall between said first and second compartment. For this purpose the inner wall may have one or more recess at the second end of the inner wall so that the channel (s) are formed between the end of the inner wall and the surface of the second closing means. Alternatively, the inner wall may have one or more openings close to the second end to provide the channel between the first and second compartment.

In another embodiment said channel(s) at the second end of said holder is/are provided in or on said second closing means. For this purpose for instance the closing means may be provided with a recess on its inside which, when the closing means is sealingly attached (for instance screwed) to the holder's second end, forms a bridging channel between the first and second compartments. Alternatively, the second closing means may be provided with a first opening at the side of the first compartment and a second opening at the side of the second compartment, and a channel in the form of a tube protruding from the outside of the second closing means between the first and the second opening of the second closing means. Through this tube the cooling liquid can flow from the first compartment to the second.

As a cooling liquid any suitable cooling liquid can be used, for instance water.

It is preferred that said first and second closing means are caps attached to the holder by means of screw bolts which are screwed into screw threads which are provided in said inner wall at the first and second end of the holder, respectively. This allows easy and water tight attachment to the holder portion of the device of the invention.

To ensure a water tight closure, sealing means such as gaskets may be provided between the first and second ends of the holder and the first and second closing caps respectively.

To enable the attachment of LED arrays on the three outer walls of the holder, each outer wall comprises one or more means for receiving one or more LED arrays on its outer surface. In principle any means suitable to attach a LED array may suffice for this purpose but it is preferred that said means for receiving a LED array comprises one or more first receptacles extending from the outer surface of a said outer wall along the longitudinal direction of said outer wall, said first receptacles having first receiving sections for a first side edge of a LED array; and one or more second receptacles extending from the outer surface of said outer wall along the longitudinal direction of said outer wall, said second receptacles having second receiving sections for a second side edge of a LED array, wherein the first and second receiving sections are mirrored and positioned opposite with respect to each other, so as to form a cage for receiving a LED array. It is preferred that the receptacles are located at the lateral ends of the outer wall so that space is optimally utilised. On each lateral side multiple receptacles may be provided. It is however preferred that each lateral side of an outer wall has only one receptacle which extends from the first end to the second end of the holder. This allows to produce the holder conveniently out of one piece, for instance by means of extrusion.

It is preferred that first and second receiving sections have an inversed L-shape configuration. By the provision of these L-shaped receiving sections a LED array can be slid easily onto the outer walls and can be easily replaced or removed from the outer wall for repair or maintenance.

Accordingly the LED lamp according to the invention can be easily provided by sliding a LED array into the cage on each outer wall. The LED arrays are arranged at the outer surface of each outer wall, and most of the length of the outer walls is covered by said on or more LED arrays. In order to be compatible with the design of the holder it is preferred that the LED arrays are elongated rectangular arrays. It is preferred that one such elongated rectangular LED array is mounted on each outer wall. Such a LED array may be a conventional array, for instance of LEDs mounted on a printed circuit board. Depending on the user's wishes one or more rows of LEDs may be provided in any suitable configuration. Means for provision of electricity to the LEDs may for instance be provided through first closing means.

In order to be used in an aqueous medium for growing organisms capable of photosynthesis, it is preferred that the LED lamp according to the invention further comprises a tubular or substantially tubular housing of light transmitting material surrounding the LED arrays in spaced relationship with the LEDs on the arrays, wherein said LED arrays extend over most of the length of said tubular housing. This light transmitting material may be of any light transmitting material such as glass or plastic. This way the LED lamps can be at least partially submerged in an aqueous liquid, such as a suspension of growing algae.

The LED lamp according to the invention can be used in a bioreactor for growing organisms capable of photosynthesis in an aqueous liquid. Such a bioreactor comprises a tank for accommodating the aqueous liquid with said organisms in it and a plurality of LED lamps according to the second aspect of the invention, wherein the LED lamps are at least partially submerged in said aqueous liquid. For sake of efficiency it is preferred that the LED lamps are fully submerged in said aqueous liquid.

The bioreactor can be provided with tubes of light transmitting material in which the LED lamps can be removably placed. Alternatively a LED lamp comprising a tubular housing of light transmitting material surrounding the LED arrays as described above can be submerged in said aqueous liquid.

In a preferred embodiment of the bioreactor the tank comprises an outer chamber and an inner chamber, wherein said inner chamber is configured within said outer chamber and in fluid connection with said outer chamber at its upper and lower ends, and means for causing a circulation of said aqueous liquid between said inner and outer chamber, wherein said LED lamps are provided in the outer chamber, and not in the inner chamber. This configuration allows for an essentially vertical circulation of growth medium. Moreover, in this setup, because the inner chamber has a small volume compared to the outer chamber, the organisms contained in the aqueous liquid will spend only a relatively short time in the dark which is advantageous for growth efficiency.

For sake of efficiency it is preferred that the outer chamber comprises multiple LED lamps extending laterally from the wall of the outer chamber over most of the length, more preferably over essentially the full length, between the wall of the outer chamber and the wall of the inner chamber. It is particularly in this embodiment that it is preferred that tubes of light transmitting material are arranged in the outer chamber and that the LED lamps can be removably placed in these tubes. This allows replacement and maintenance of LED lamps without the necessity of emptying the tank.

In a further preferred configuration of the bioreactor the outer chamber is defined by a first vertical cylinder and the inner chamber is defined by a second vertical cylinder, wherein the second vertical cylinder is mounted into said first vertical cylinder. This way it is possible to produce a circulation of growth medium with an upward flow through the outer cylinder and a downward flow through the inner cylinder or vice versa.

The operation of the bioreactor will be discussed in more detail on the basis of the steps of the method of the invention.

In this method a flow of said aqueous liquid containing organisms capable of biosynthesis is provided, and the organisms in the flowing aqueous liquid are exposed to a plurality of LED lamps according to the invention, wherein the LED lamps are at least partially submerged in said aqueous liquid.

It is preferred that this method is performed in a bioreactor according to the invention, comprising providing a circulation between said outer and inner chamber, wherein the direction of flow in said inner chamber is inverse to the direction of the flow in said outer chamber. Such a flow can be caused by a pump or, more preferably by bubbling gas, such as air into one of the outer or inner chamber. This can be realised by providing a bubble generating means on the lower side of one of the chambers. This causes a local decrease in the density of the growth medium causing an upward flow. As soon as the bubbles have reached the upper surface the gas contained in the bubbles will be released causing the density of the medium to increase. As a result the denser medium will enter the chamber which is not provided at its bottom with a bubble generating means and the denser medium will flow downwards through this chamber. The bottom of the inner chamber is on both sides in fluid contact with the medium in the outer chamber so that the down flowing medium that has reached the bottom of the chamber will enter the chamber which is provided with said bubble generating means so that the medium is forced to flow up again. This way a circular flow is created. The inner chamber is preferably fully fit into the outer chamber and fully submerged in the growth medium.

In a particularly preferred embodiment the direction of flow in said outer chamber is upwards, while the direction of flow in said inner chamber is downwards. For this purpose bubble generating means are provided at the bottom of the outer chamber at a distance from the inner chamber. Bubbles are preferably released in a bubbling releasing zone extending around the inner chamber at a distance from it. The bubbles cause an upward flow of the growth medium in the outer chamber, thus passing the organisms capable of photosynthesis along the LED lamps which are arranged in the outer chamber so that they are exposed to the light. Once bubbles have reached the surface, the gas contained in said bubbles is released. The medium's density increased and the medium flows downwards through the inner chamber until at the bottom it flows back into the outer chamber, where new bubbles are blown into the medium causing the medium to flow upwards again through the outer chamber.

In principle the method is suitable for all kinds of organisms which are capable of photosynthesis and growing in an aqueous medium. It is preferred that the organisms are algae, because algae produce lipids, vegetable oils and proteins which can be used for various purposes such as a source for human or animal nutrition or biofuel.

### Detailed description of the drawings

The invention will now be further elucidated in the attached drawings. The following explanation is meant to illustrate and explain the invention and not to limit the claims.

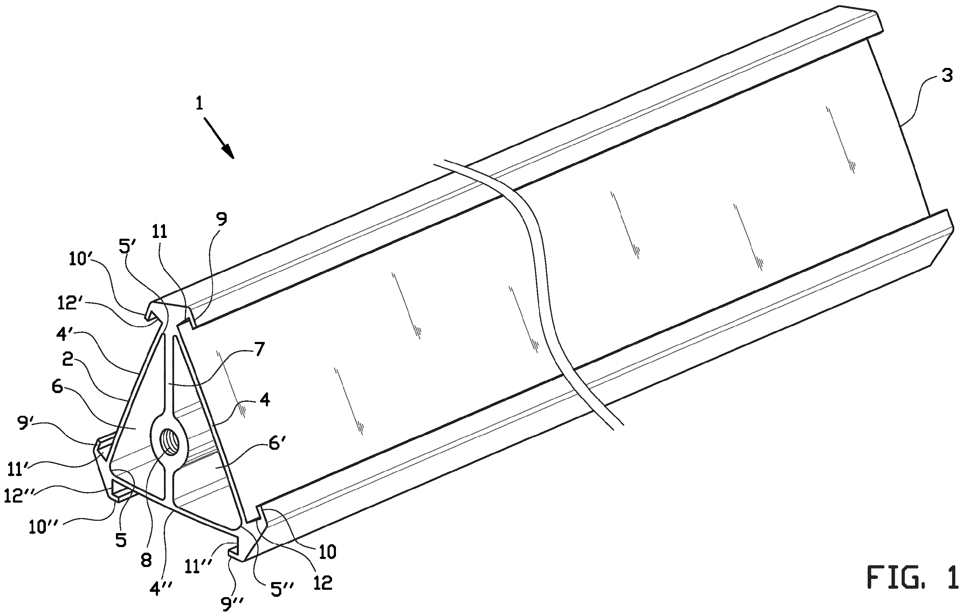

Fig. 1 shows a holder 1 of a carrier device according to the invention. Holder 1 has a first end 2 and a second end 3. It has three elongated outer walls 4, 4', 4" extending between the first 2 and second end 3, wherein each wall is laterally connected to the other two walls by a water impermeable connection 5, 5', 5". As can be seen in Fig. 1 the outer walls 4, 4', 4" are oriented with respect to each other to define a space of substantially equilateral triangular cross section, which is divided into two hollow compartments 6 and 6' by an inner wall 7 extending between said first and said second end and extending perpendicular from the inner surface of one of the outer walls to the opposite inner angle position between the other outer walls. This way two compartments 6 and 6' of equal volume are provided. In the inner wall 7 screw threads 8 are provided at the first and second end 2, 3 of the holder 1. The three outer walls 4, 4', 4" of the holder are each provided with a first receptacle 9, 9', 9" and second receptacle 10, 10', 10" extending from the outer surface of a said outer wall along the longitudinal direction of said outer wall, each receptacle extending from the first end 2 to the second end 3 of the holder. The first and second receptacles 9, 9', 9", 10, 10', 10" are arranged at the lateral ends of the outer wall so that space is optimally utilised. The first and second receptacles have first and second receiving sections 11, 11', 11" and 12, 12', 12", respectively, which have an inversed L-shape configuration.

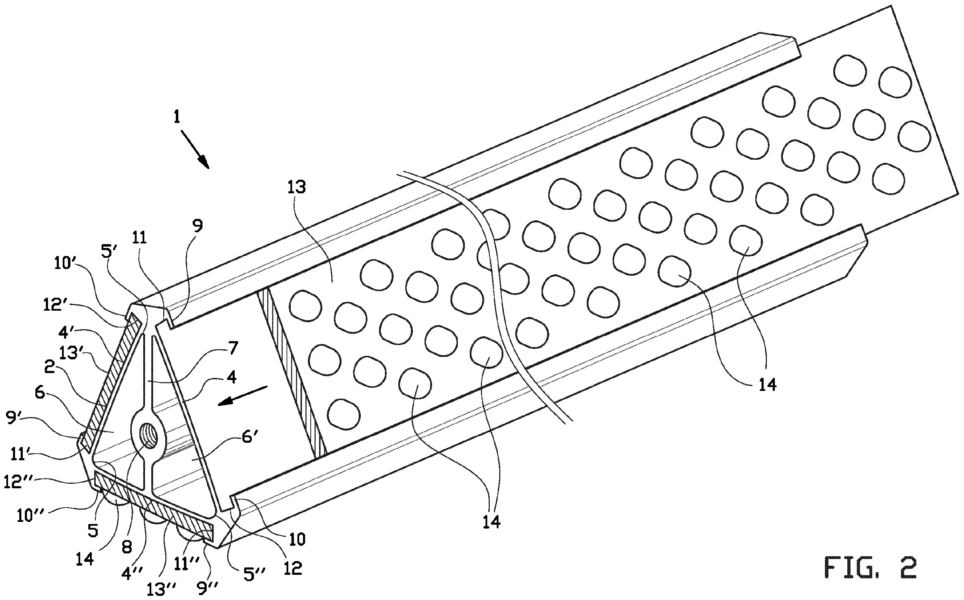

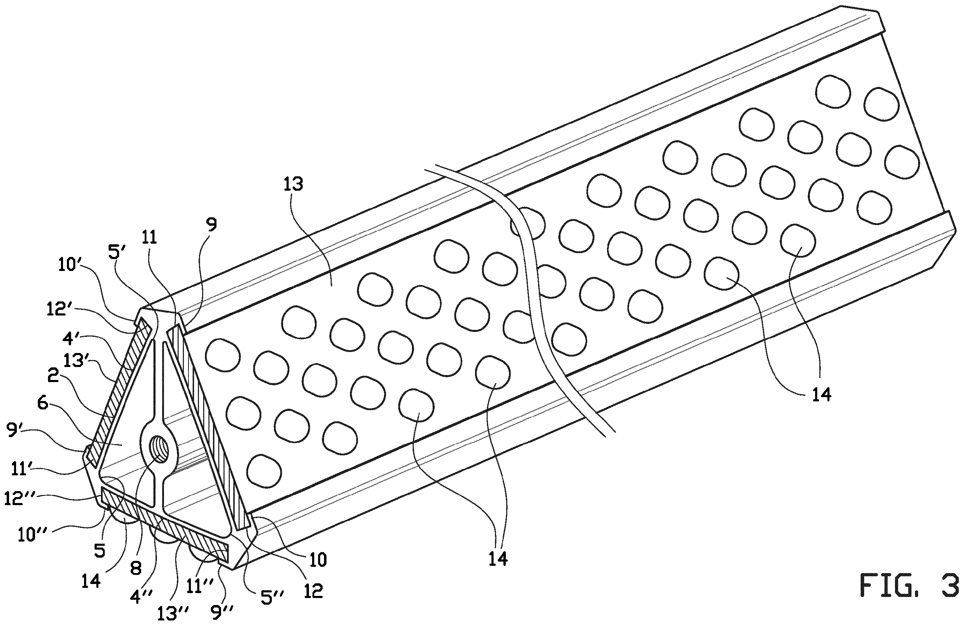

As shown in Figs. 2 and 3, by the provision of these L-shaped receiving sections 11, 11', 11" and 12, 12', 12" LED arrays 13, 13', 13" comprising multiple rows of LEDs 14 can be slid easily (see arrow) onto the outer walls and can be easily replaced or removed from the outer wall for repair or maintenance.

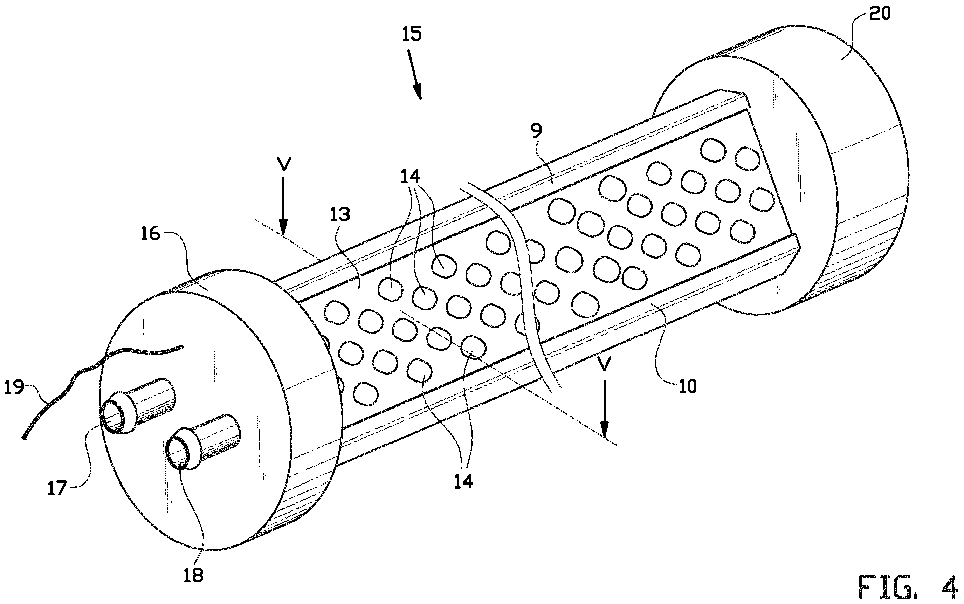

Fig. 4 shows an exemplary embodiment of the LED lamp of the invention 15. In this embodiment a first cap 16 is screwed onto the first end of the holder 1. Said first cap 16 comprises an inlet 17 which allows the introduction of a cooling liquid into the first hollow compartment 6, and an outlet 18 for passing a cooling liquid out of the second hollow compartment 6'. A cable 19 for providing electricity to the LEDs can be provided through first cap 17. On the second end 3 of the holder 1 a second cap 20 is screwed onto the holder.

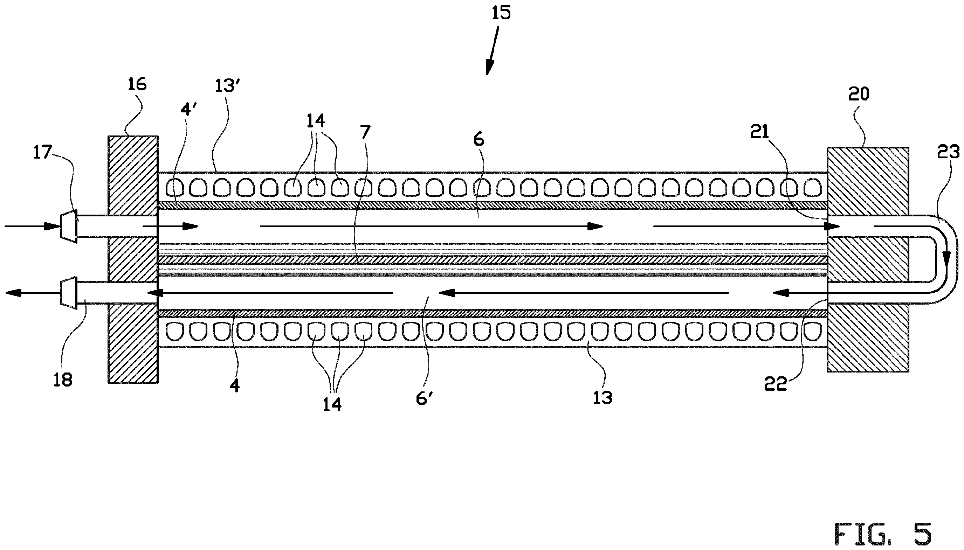

Referring now to Fig. 5, which shows the a cross section of the LED lamp of Fig. 4 in the longitudinal direction at the position of line v, the second cap 20 is provided with a first opening 21 at the side of the first hollow compartment 6 and a second opening 22 at the side of the second hollow compartment 6', and a tube 23 protruding from the outside of the second cap 20 between the first 21 and the second opening 22. The provision of tube 23 allows a fluid connection between the first and second hollow compartment (6, 6'). When in operation the LEDs 14 of this exemplary LED lamp 15 are cooled as follows. A cooling liquid is introduced via inlet 17 into first hollow compartment 6. The direction of the flowing cooling liquid is indicated with arrows. The cooling liquid passes through hollow compartment 6 while cooling the LEDs 14 of LED array 13' mounted on outer walls 4' and 4". At the second end of the holder 1 the cooling liquid enters opening 21 in second lid 20 and is allowed to flow via tube 23 and opening 22 into hollow compartment 6'. The cooling liquid passes through hollow compartment 6' while cooling the LEDs 14 of LED array 13 mounted on outer walls 4 and 4". The cooling liquid leaves the second hollow compartment 6' via outlet 18. The triangular arrangement of the holder 1 with the longitudinal axes of the arrays of LEDs spaced apart 120° provides for an arrangement for optimal cooling of the LEDs 14 mounted on the holder.

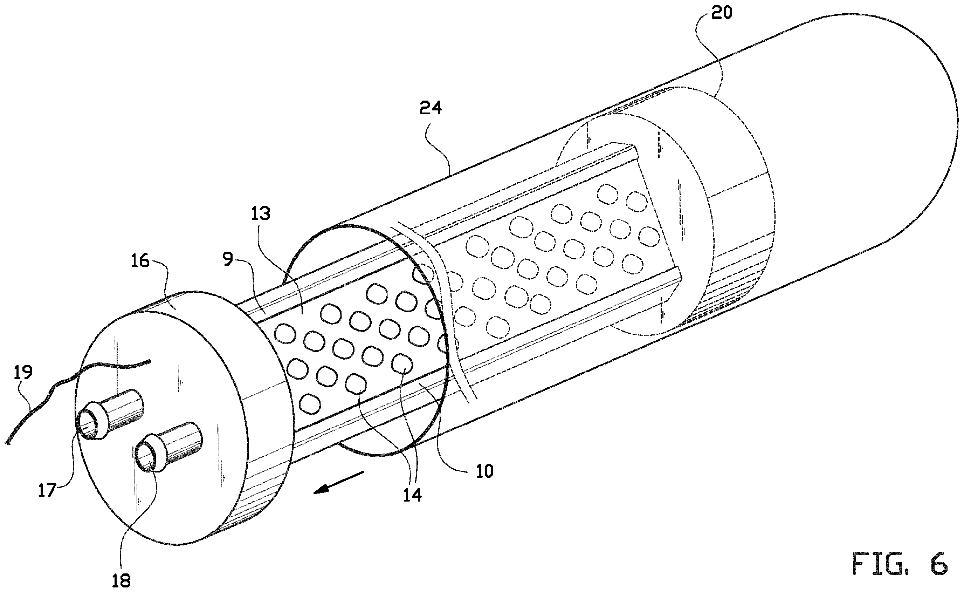

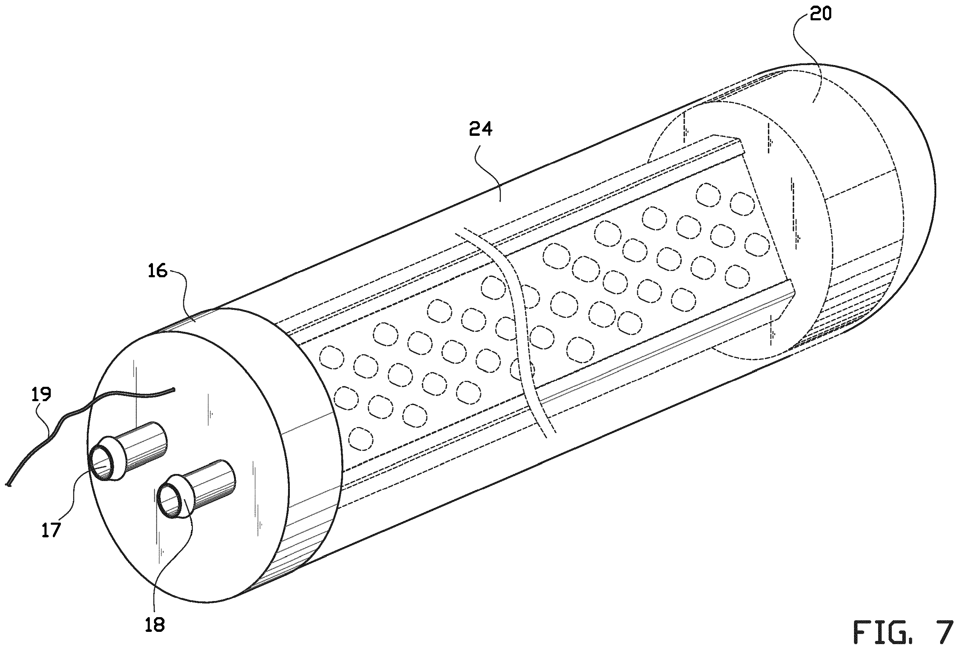

As shown in Fig. 6, the LED lamp of Fig. 5 can be slid into a tubular housing 24 of light transmitting material surrounding the LED arrays 13, 13', 13" in spaced relationship with the LEDs 14 on the arrays. This tubular housing allows the LED lamps to be at least partially submerged in an aqueous liquid such as a suspension of growing algae. Fig. 7 shows the LED arrays 13, 13', 13" extending over most of the length of said tubular housing 24.

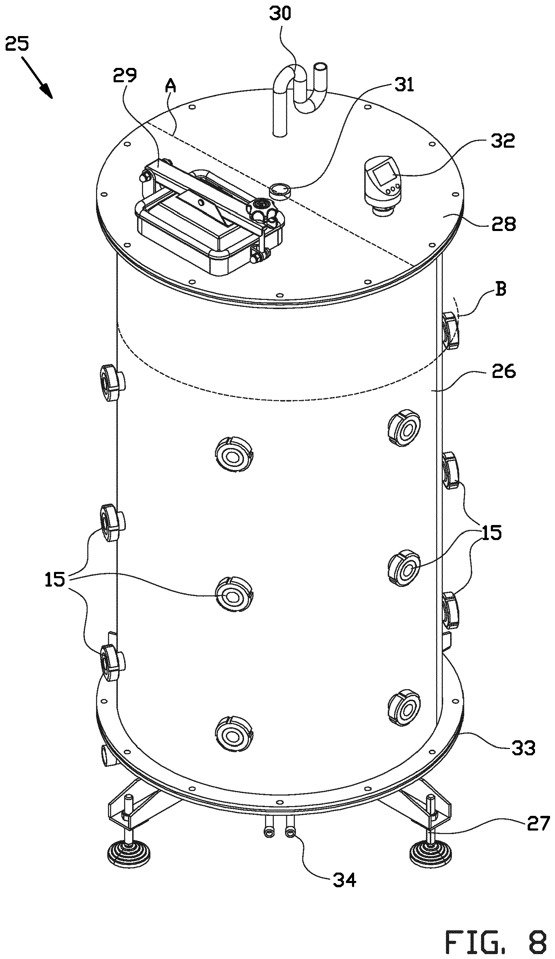

Fig. 8 shows an exemplary embodiment of a bioreactor 25 in accordance with the invention. This bioreactor comprises a cylindrical tank 26 and multiple LED lamps 15 extend laterally from the outside of the tank wall into the tank. In this embodiment the tank stands on four feet 27. The tank 26 is closed with a lid 28 onto which means 29 for opening and closing the tank are provided. On the lid of this bioreactor further a pipe 30 for degassing, a sealable pipe 31 for water supply, and a water level detector 32 are provided. At the bottom of the tank a bottom plate 33 is provided. Via this bottom plate gas can be provided into the tank via pipe 34.

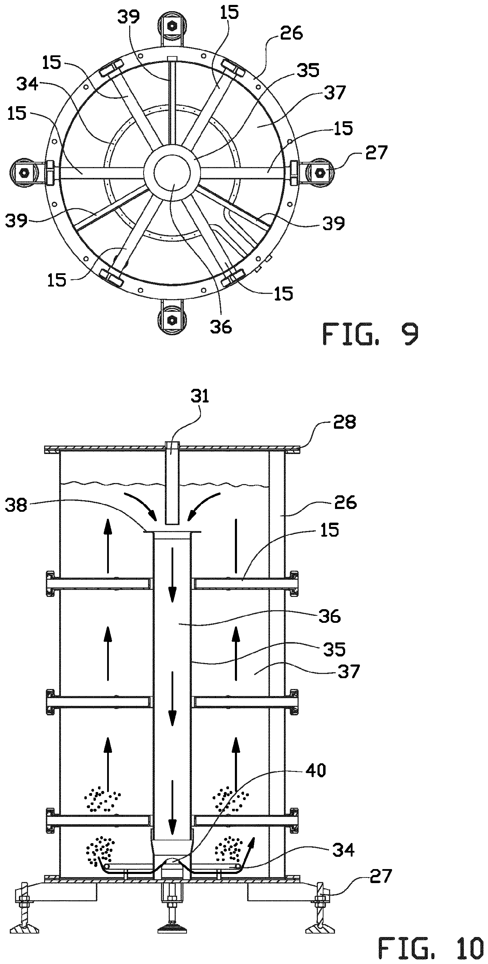

Figs. 9 and 10 show the inside of the bioreactor according to Fig. 8. Fig. 9 shows a cross section along line B of the bioreactor of Fig. 8. Fig. 10 shows a cross section along line A of the bioreactor of Fig. 8 in case the tank of the bioreactor is filled with liquid growth medium. The bioreactor of Figs. 8-10 allows performing a preferred embodiment of the method according to the invention wherein algae are grown by creating a circulation of algae along the LED lamps submerged in the growth medium of the algae in the reactor. Inside tank 26 an inner cylinder 35 is placed supported by supporting members 39. The inner cylinder 35 defines an inner chamber 36. On the other hand the cylindrical tank 26 defines an outer chamber 37 surrounding inner chamber 36. When in operation the tank is filled with an aqueous growth medium containing algae. The level of growth medium should be such that the full inner chamber is submerged in the growth medium. To ensure that the level of growth medium is maintained at acceptable or optimal levels the tank 26 is provided with sealable pipe 31 for water supply, and a water level detector 32. Flow of the growth medium is effected by the provision of pipe 34 which extends on the bottom of the tank in a circumferential fashion around the inner cylinder at a suitable distance from it to provide a means for introducing gas such as air into the outer chamber 37. This causes a local decrease in the density of the growth medium causing an upward flow (see arrows for the direction of flow). During the way up the algae in the growth medium are exposed to the light emitted by LED lamps 15 which are provided with a tubular housing 24 and which extend in the growth medium laterally from the wall of the tank 26 to the inner cylinder 35 and which are evenly distributed throughout the outer chamber 37. As soon as the bubbles have reached the upper surface the gas contained in the bubbles will be released on the surface of the growth medium, causing the density of the growth medium to increase locally. As a result the denser medium will enter the inner chamber 36 via opening 38. Because of its higher density the medium flows downwards. The growth medium leaves the inner chamber via bottom outlets 40 and enters the outer chamber 37. There the medium is exposed again to gas bubbles released by pipe 34, causing the medium to flow up again in the outer chamber 37, thus effecting a circulation.

## Claims

1. A carrier device for LED arrays, comprising a holder (1) which comprises:
a first end (2) and a second end (3);
three elongated outer walls (4, 4', 4") extending between the first and second end (2, 3), wherein each wall is laterally connected to the other two walls by a water impermeable connection (5, 5', 5"), and wherein the walls are oriented with respect to each other to define a space of substantially equilateral triangular cross section; and
a first and second hollow compartment (6, 6') within said space,
wherein each outer wall comprises one or more means for receiving a LED array (13) on its outer surface, wherein at said first end (2) a first closing means (16) is provided and at said second end (3) a second closing means (20) is provided,
wherein said first closing means comprises an inlet (17) for introducing a cooling liquid into said first hollow compartment (6), and an outlet (18) for passing said cooling liquid out of said second hollow compartment (6');
wherein at the second end (3) of said holder one or more channels are provided which allow flow of said cooling liquid between the first hollow compartment (6) and the second hollow compartment (6').

2. The carrier device according to claim 1, wherein said first and second hollow compartments (6, 6') are defined by an inner wall (7) extending between said first and said second end (2, 3) and extending perpendicular from the inner surface of one of the outer walls (4, 4', 4") to the opposite inner angle position between the other outer walls, wherein the connection between said inner wall (7) and said inner surface and inner angle position is water impermeable.

3. The carrier device according to claim 2, wherein said channel at the second end (3) of said holder (1) is an opening in said inner wall (7) between said first and second compartments (6, 6'); or
wherein said channel (23) at the second end (3) of said holder is provided in or on said second closing means (20).

4. The carrier device according to any of claims 1 to 3, wherein said first and second closing means are caps (16, 17) attached to the holder by means of screw bolts which are screwed into screw threads (8) which are provided in said inner wall (7) at the first and second end (2, 3) of the holder, respectively.

5. The carrier device according to any of the claims 1 to 4, wherein said means for receiving a LED array comprises one or more first receptacles (9, 9', 9",) extending from the outer surface of a said outer wall along the longitudinal direction of said outer wall, said first receptacles having first receiving sections (11, 11', 11") for a first side edge of a LED array (13, 13', 13"); and one or more second receptacles (10, 10', 10") extending from the outer surface of said outer wall along the longitudinal direction of said outer wall, said second receptacles having second receiving sections (12, 12' 12") for a second side edge of a LED array (13, 13', 13"), wherein the first and second receiving sections are mirrored and positioned opposite with respect to each other, so as to form a cage for receiving a LED array.

6. A LED lamp (15), comprising the carrier device according to any of claims 1 to 5, and at least one LED array (13, 13', 13") arranged at the outer surface of each outer wall (4, 4', 4"), wherein most of the length of the outer walls is covered by said LED arrays.

7. The LED lamp (15) according to claim 6, further comprising a substantially tubular housing (24) of light transmitting material surrounding the LED arrays (13, 13', 13") in spaced relationship with the LEDs (14) on the arrays, wherein said LED arrays extend over most of the length of said tubular housing.

8. A bioreactor (25) for growing organisms capable of photosynthesis in an aqueous liquid, comprising
a tank (26) for accommodating the aqueous liquid with said organisms in it; and
a plurality of LED lamps (15) according to any of the claims 6 and 7, wherein the LED lamps are at least partially submerged in said aqueous liquid.

9. The bioreactor according to claim 8, wherein said tank comprises
an outer chamber (37) and an inner chamber (36), wherein said inner chamber (36) is configured within said outer chamber (37) and in fluid connection with said outer chamber (37) at its upper and down ends, and
means for causing a circulation of said aqueous liquid between said inner and outer chamber,
wherein said LED lamps (15) are provided in the outer chamber, and not in the inner chamber.

10. The bioreactor according to claim 9, wherein the outer chamber comprises multiple LED lamps (15) according to claim 7, extending laterally from the wall of the outer chamber (37) over essentially the full length between the wall of the outer chamber and the wall of the inner chamber.

11. The bioreactor according to any of the claims 8 to 10, wherein the outer chamber (37) is defined by a first vertical cylinder and the inner chamber (36) is defined by a second vertical cylinder, wherein the second vertical cylinder is mounted into said first vertical cylinder.

12. A method for growing organisms capable of photosynthesis in an aqueous liquid, comprising
providing a flow of said aqueous liquid, and
exposing the organisms in the flowing aqueous liquid to a plurality of LED lamps (15) according to any of the claims 6 and 7, wherein the LED lamps (15) are at least partially submerged in said aqueous liquid.

13. The method according to claim 12, which is performed in a bioreactor (25) according to any of the claims 9 to 11, comprising providing a circulation between said outer and inner chamber, wherein the direction of flow in said inner chamber is inverse to the direction of the flow in said outer chamber.

14. The method according to claim 13, wherein the direction of flow in said outer chamber (37) is upwards, and wherein the direction of flow in said inner chamber (36) is downwards.

## Patentansprüche

1. Trägervorrichtung für LED-Arrays, umfassend einen Halter (1), welcher umfasst:
ein erstes Ende (2) und ein zweites Ende (3);
drei langgestreckte Außenwände (4, 4', 4"), welche sich zwischen dem ersten und dem zweiten Ende (2, 3) erstrecken, wobei jede Wand seitlich mit den anderen beiden Wänden über eine wasserundurchlässige Verbindung (5, 5', 5") verbunden ist, und wobei die Wände bezüglich einander orientiert sind, um einen Raum von im Wesentlichen gleichseitigem dreieckigem Querschnitt zu definieren; und
ein erstes und ein zweites hohles Abteil (6, 6') innerhalb des Raums,
wobei jede Außenwand ein oder mehrere Mittel zur Aufnahme eines LED-Arrays (13) an ihrer äußeren Oberfläche umfasst, wobei an dem ersten Ende (2) ein erstes Verschlussmittel (16) bereitgestellt ist und an dem zweiten Ende (3) ein zweites Verschlussmittel (20) bereitgestellt ist,
wobei das erste Verschlussmittel einen Einlass (17) zum Einführen einer Kühlflüssigkeit in das erste hohle Abteil (6) und einen Auslass (18) zum Heraustretenlassen der Kühlflüssigkeit aus dem zweiten hohlen Abteil (6') umfasst;
wobei an dem zweiten Ende (3) des Halters ein oder mehrere Kanäle bereitgestellt sind, welche eine Strömung der Kühlflüssigkeit zwischen dem ersten hohlen Abteil (6) und dem zweiten hohlen Abteil (6') zulassen.

2. Trägervorrichtung nach Anspruch 1, wobei das erste und das zweite hohle Abteil (6, 6') durch eine Innenwand (7) definiert sind, welche sich zwischen dem ersten und dem zweiten Ende (2, 3) erstreckt und sich senkrecht von der inneren Oberfläche einer der Außenwände (4, 4', 4") bis zu der gegenüberliegenden Innenwinkelposition zwischen den anderen Außenwänden erstreckt, wobei die Verbindung zwischen der Innenwand (7) und der inneren Oberfläche und der Innenwinkelposition wasserundurchlässig ist.

3. Trägervorrichtung nach Anspruch 2, wobei der Kanal an dem zweiten Ende (3) des Halters (1) eine Öffnung in der Innenwand (7) zwischen dem ersten und dem zweiten Abteil (6, 6') ist; oder
wobei der Kanal (23) an dem zweiten Ende (3) des Halters in oder an dem zweiten Verschlussmittel (20) bereitgestellt ist.

4. Trägervorrichtung nach einem der Ansprüche 1 bis 3, wobei das erste und das zweite Verschlussmittel (16, 17) Kappen sind, welche an den Halter mittels Schraubenbolzen angebunden sind, die in Schraubgewinde (8) eingeschraubt sind, welche in der Innenwand (7) an dem ersten bzw. zweiten Ende (2, 3) des Halters bereitgestellt sind.

5. Trägervorrichtung nach einem der Ansprüche 1 bis 4, wobei das Mittel zum Aufnehmen eines LED-Arrays eine oder mehrere erste Aufnahmen (9, 9', 9") umfasst, welche sich von der äußeren Oberfläche einer der Außenwände entlang der Längsrichtung der Außenwand erstrecken, wobei die ersten Aufnahmen erste Aufnahmeabschnitte (11, 11' 11") für eine erste Seitenkante eines LED-Arrays (13, 13', 13") aufweisen; und eine oder mehrere zweite Aufnahmen (10, 10', 10"), welche sich von der äußeren Oberfläche der Außenwand entlang der Längsrichtung der Außenwand erstrecken, wobei die zweiten Aufnahmen zweite Aufnahmeabschnitte (12, 12', 12") für eine zweite Seitenkante eines LED-Arrays (13, 13', 13") aufweisen, wobei die ersten und die zweiten Aufnahmeabschnitte gespiegelt und gegenüberliegend zueinander positioniert sind, um einen Käfig zur Aufnahme eines LED-Arrays zu bilden.

6. LED-Lampe (15), umfassend die Trägervorrichtung nach einem der Ansprüche 1 bis 5, und mindestens ein LED-Array (13, 13', 13"), welches an der äußeren Oberfläche jeder Außenwand (4, 4', 4") angeordnet ist, wobei der größte Teil der Länge der Außenwände durch die LED-Arrays abgedeckt ist.

7. LED-Lampe (15) nach Anspruch 6, ferner umfassend ein im Wesentlichen röhrenförmiges Gehäuse (24) aus einem lichtdurchlässigen Material, welches die LED-Arrays (13, 13', 13") mit Abstand zu den LEDs (14) auf den Arrays umgibt, wobei sich die LED-Arrays über den größten Teil der Länge des röhrenförmigen Gehäuses erstrecken.

8. Bioreaktor (25) zum Wachsenlassen von zur Photosynthese fähigen Organismen in einer wässrigen Flüssigkeit, umfassend
einen Tank (26) zum Aufnehmen der wässrigen Flüssigkeit mit den Organismen darin; und
eine Mehrzahl von LED-Lampen (15) nach einem der Ansprüche 6 und 7, wobei die LED-Lampen mindestens teilweise in die wässrige Flüssigkeit eingetaucht sind.

9. Bioreaktor nach Anspruch 8, wobei der Tank umfasst
eine äußere Kammer (37) und eine innere Kammer (36), wobei die innere Kammer (36) innerhalb der äußeren Kammer (37) ausgebildet ist und an ihrem oberen und unteren Ende in Fluidverbindung mit der äußeren Kammer (37) steht, und
Mittel zum Bewirken einer Zirkulation der wässrigen Flüssigkeit zwischen der inneren und der äußeren Kammer,
wobei die LED-Lampen (15) in der äußeren und nicht in der inneren Kammer bereitgestellt sind.

10. Bioreaktor nach Anspruch 9, wobei die äußere Kammer mehrere LED-Lampen (15) nach Anspruch 7 umfasst, welche sich seitlich von der Wand der äußeren Kammer (37) über im Wesentlichen die gesamte Länge zwischen der Wand der äußeren Kammer und der Wand der inneren Kammer erstrecken.

11. Bioreaktor nach einem der Ansprüche 8 bis 10, wobei die äußere Kammer (37) durch einen ersten vertikalen Zylinder definiert ist und die innere Kammer (36) durch einen zweiten vertikalen Zylinder definiert ist, wobei der zweite vertikale Zylinder in den ersten vertikalen Zylinder montiert ist.

12. Verfahren zum Wachsenlassen von zur Photosynthese fähigen Organismen in einer wässrigen Flüssigkeit, umfassend
Bereitstellen einer Strömung der wässrigen Flüssigkeit und
Aussetzen der Organismen in der strömenden wässrigen Flüssigkeit einer Mehrzahl von LED-Lampen (15) nach einem der Ansprüche 6 und 7, wobei die LED-Lampen (15) mindestens teilweise in die wässrige Flüssigkeit eingetaucht sind.

13. Verfahren nach Anspruch 12, welches in einem Bioreaktor (25) nach einem der Ansprüche 9 bis 11 durchgeführt wird, umfassend ein Bereitstellen einer Zirkulation zwischen der äußeren und der inneren Kammer, wobei die Strömungsrichtung in der inneren Kammer umgekehrt zu der Strömungsrichtung in der äußeren Kammer ist.

14. Verfahren nach Anspruch 13, wobei die Strömungsrichtung in der äußeren Kammer (37) aufwärts gerichtet ist und wobei die Strömungsrichtung in der inneren Kammer (36) abwärts gerichtet ist.

## Revendications

1. Dispositif porteur pour des barrettes de DEL, comprenant un support (1) qui comprend :
une première extrémité (2) et une seconde extrémité (3) ;
trois parois extérieures allongées (4, 4', 4") s'étendant entre les première et seconde extrémités (2, 3), dans lequel chaque paroi est raccordée latéralement aux deux autres parois par un raccordement imperméable à l'eau (5, 5', 5"), et dans lequel les parois sont orientées les unes par rapport aux autres pour définir un espace de section transversale triangulaire sensiblement équilatérale ; et
un premier et un second compartiment creux (6, 6') au sein dudit espace,
dans lequel chaque paroi extérieure comprend un ou plusieurs moyens pour recevoir une barrette de DEL (13) sur sa surface extérieure, dans lequel à ladite première extrémité (2) un premier moyen de fermeture (16) est fourni et à ladite seconde extrémité (3) un second moyen de fermeture (20) est fourni,
dans lequel ledit premier moyen de fermeture comprend un orifice d'entrée (17) pour introduire un liquide de refroidissement dans ledit premier compartiment creux (6), et un orifice de sortie (18) pour faire passer ledit liquide de refroidissement hors dudit second compartiment creux (6') ;
dans lequel à la seconde extrémité (3) dudit support un ou plusieurs canaux sont fournis qui permettent un écoulement dudit liquide de refroidissement entre le premier compartiment creux (6) et le second compartiment creux (6').

2. Dispositif porteur selon la revendication 1, dans lequel lesdits premier et second compartiments creux (6, 6') sont définis par une paroi intérieure (7) s'étendant entre ladite première et ladite seconde extrémité (2, 3) et s'étendant perpendiculairement depuis la surface intérieure de l'une des parois extérieures (4, 4', 4") vers la position d'angle intérieure opposée entre les autres parois extérieures, dans lequel le raccordement entre ladite paroi intérieure (7) et lesdites surface intérieure et position d'angle intérieure est imperméable à l'eau.

3. Dispositif porteur selon la revendication 2, dans lequel ledit canal à la seconde extrémité (3) dudit support (1) est une ouverture dans ladite paroi intérieure (7) entre lesdits premier et second compartiments (6, 6') ; ou
dans lequel ledit canal (23) à la seconde extrémité (3) dudit support est fourni dans ou sur ledit second moyen de fermeture (20).

4. Dispositif porteur selon l'une quelconque des revendications 1 à 3, dans lequel lesdits premier et second moyens de fermeture sont des capuchons (16, 17) attachés au support au moyen de boulons à vis qui sont vissés dans des filets de vis (8) qui sont fournis dans ladite paroi intérieure (7) aux première et seconde extrémités (2, 3) du support, respectivement.

5. Dispositif porteur selon l'une quelconque des revendications 1 à 4, dans lequel lesdits moyens pour recevoir une barrette de DEL comprennent un ou plusieurs premiers réceptacles (9, 9', 9"), s'étendant depuis la surface extérieure d'une dite paroi extérieure suivant la direction longitudinale de ladite paroi extérieure, lesdits premiers réceptacles ayant des premières sections de réception (11, 11', 11") pour un premier bord de côté d'une barrette de DEL (13, 13', 13") ; et un ou plusieurs seconds réceptacles (10, 10', 10") s'étendant depuis la surface extérieure de ladite paroi extérieure suivant la direction longitudinale de ladite paroi extérieure, lesdits seconds réceptacles ayant des secondes sections de réception (12, 12', 12") pour un second bord de côté d'une barrette de DEL (13, 13', 13"), dans lequel les première et seconde sections de réception sont placées en symétrie et positionnées opposées l'une par rapport à l'autre, de façon à former une cage pour recevoir une barrette de DEL.

6. Lampe à DEL (15), comprenant le dispositif porteur selon l'une quelconque des revendications 1 à 5, et au moins une barrette de DEL (13, 13', 13") agencée au niveau de la surface extérieure de chaque paroi extérieure (4, 4', 4"), dans laquelle la plus grande partie de la longueur des parois extérieures est couverte desdites barrettes de DEL.

7. Lampe à DEL (15) selon la revendication 6, comprenant en outre un logement (24) sensiblement tubulaire en matériau transmettant la lumière entourant les barrettes de DEL (13, 13', 13") en relation espacée avec les DEL (14) sur les barrettes, dans laquelle lesdites barrettes de DEL s'étendent sur la plus grande partie de la longueur dudit logement tubulaire.

8. Bioréacteur (25) pour cultiver des organismes capables de photosynthèse dans un liquide aqueux, comprenant
un réservoir (26) pour accueillir le liquide aqueux avec lesdits organismes dedans ; et
une pluralité de lampes à DEL (15) selon l'une quelconque des revendications 6 et 7, dans lequel les lampes à DEL sont immergées au moins partiellement dans ledit liquide aqueux.

9. Bioréacteur selon la revendication 8, dans lequel ledit réservoir comprend
une chambre extérieure (37) et une chambre intérieure (36), dans lequel ladite chambre intérieure (36) est configurée au sein de ladite chambre extérieure (37) et en raccordement fluidique avec ladite chambre extérieure (37) à ses extrémités supérieure et inférieure, et
un moyen pour provoquer une circulation dudit liquide aqueux entre lesdites chambres intérieure et extérieure,
dans lequel lesdites lampes à DEL (15) sont fournies dans la chambre extérieure, et pas dans la chambre intérieure.

10. Bioréacteur selon la revendication 9, dans lequel la chambre extérieure comprend de multiples lampes à DEL (15) selon la revendication 7, s'étendant latéralement depuis la paroi de la chambre extérieure (37) essentiellement sur toute la longueur entre la paroi de la chambre extérieure et la paroi de la chambre intérieure.

11. Bioréacteur selon l'une quelconque des revendications 8 à 10, dans lequel la chambre extérieure (37) est définie par un premier cylindre vertical et la chambre intérieure (36) est définie par un second cylindre vertical, dans lequel le second cylindre vertical est monté dans ledit premier cylindre vertical.

12. Méthode pour cultiver des organismes capables de photosynthèse dans un liquide aqueux, comprenant
la fourniture d'un écoulement dudit liquide aqueux, et
l'exposition des organismes dans le liquide aqueux en écoulement à une pluralité de lampes à DEL (15) selon l'une quelconque des revendications 6 et 7, dans laquelle les lampes à DEL (15) sont immergées au moins partiellement dans ledit liquide aqueux.

13. Méthode selon la revendication 12, qui est réalisée dans un bioréacteur (25) selon l'une quelconque des revendications 9 à 11, comprenant la fourniture d'une circulation entre lesdites chambres extérieure et intérieure, dans laquelle la direction d'écoulement dans ladite chambre intérieure est l'inverse de la direction de l'écoulement dans ladite chambre extérieure.

14. Méthode selon la revendication 13, dans laquelle la direction d'écoulement dans ladite chambre extérieure (37) est vers le haut, et dans laquelle la direction d'écoulement dans ladite chambre intérieure (36) est vers le bas.
